# FASCICULE DE BREVET EUROPEEN

(11) **EP 1 449 551 B1**
(45) Date de publication et mention de la délivrance du brevet: **06.04.2011**
(21) Numéro de dépôt: 03356032.7
(22) Date de dépôt: 24.02.2003
(51) Int. Cl.: A61M 5/00, B65D 25/10, B01L 9/00

(54) **Ensemble d'éléments pour le groupage de corps de seringue**
Vorrichtung zur Gruppierung von Spritzen
Device for grouping syringes

(43) Date de publication de la demande: 25.08.2004
(73) Titulaire: Becton Dickinson France, 38800 Le Pont de Claix (FR)
(72) Inventeur: Vedrine, Lionel, 38400 Saint Martin d'Heres (FR); Cocheteux, Bruno, 38500 Voiron (FR)
(74) Mandataire: Guerre, Dominique

(56) Documents cités:
- WO-A-99/45984
- BE-A- 741 280
- US-A- 3 606 006
- US-A- 5 897 532

## Description

La présente invention concerne un ensemble d'éléments pour le groupage de corps de seringue.

Il est fréquent que des corps de seringue soient fabriqués sur un site et remplis sur un autre site, ce qui implique un transport de ces corps de seringue d'un site à l'autre.

Pour ce transport, et pour éviter toute détérioration des corps de seringue, il est courant de grouper les corps de seringue sur un plateau à cheminées de réception des corps de seringue, puis de placer l'ensemble dans une boîte d'emballage, cette boîte étant ensuite scellée et stérilisée. A destination, la boîte est ouverte puis le plateau est extrait de cette boîte, ce plateau pouvant être utilisé pour la manipulation des corps de seringue et le remplissage de ceux-ci par des moyens automatisés.

Un plateau de groupage connu, utilisé dans cette application spécifique, comprend une plaque de base dans laquelle est formée une pluralité de cheminées faisant saillie d'une face de cette plaque de base, ces cheminées étant dimensionnées pour recevoir les corps de seringue à travers elles jusqu'à venue des collerettes proximales que comprennent les corps de seringue en appui contre les bords libres supérieurs de ces cheminées.

Un plateau de ce type a pour inconvénient de ne pas présenter une rigidité très importante, ce qui limite ses dimensions et donc le nombre de corps de seringue qu'il est possible de placer dans une même boîte. Cette absence de rigidité peut également avoir des conséquences néfastes sur les opérations automatisées ou manuelles de manipulation des plateaux, d'engagement ou d'extraction des plateaux dans ou hors des boîtes d'emballage, et de remplissage des corps de seringue.

De plus, les corps de seringue ne sont pas immobilisés par rapport au plateau, tant dans le sens de leur axe longitudinal qu'en pivotement autour de cet axe. L'absence d'immobilisation axiale limite les manipulations possibles de la boîte d'emballage, lors des opérations de stérilisation/décontamination de cette boîte, ou les positions de stockage possibles de cette boîte, ou oblige à un maintien des corps de seringue par rapport au plateau lors de manipulations ou de positions de stockage impliquant un retournement de la boîte. Les manipulations ou positions de stockage possibles du plateau avant ou après mise en place dans cette boîte sont également limitées de la même manière. L'absence d'immobilisation en pivotement oblige à prévoir une distance suffisante entre deux cheminées adjacentes pour que les collerettes des corps de seringue reçus dans ces deux cheminées, lorsque ces collerettes ne sont pas circulaires, ne viennent pas en contact l'une de l'autre, ce qui pourrait les détériorer ou générer des particules. Il en résulte une limitation du nombre de corps de seringue qu'il est possible de prévoir sur un plateau de dimensions données.

En outre, le plateau existant ne permet pas la mise en place sur les corps de seringue de pièces proximales de prise d'appui facilitant la prise d'appui des doigts de l'utilisateur au moment de la réalisation d'une injection. Or, il serait intéressant de pouvoir mettre de telles pièces proximales de prise d'appui en place sur les corps de seringue avant remplissage de ces corps de seringue, tout en assurant une parfaite protection de ces pièces proximales de prise d'appui lors du transport des corps de seringue.

Le document BE 741280 décrit un plateau pour le groupage d'objets, ledit plateau comprenant des renflements servant à limiter l'inclinaison latérale des objets.

Le document WO 99/45984 décrit un plateau pour le groupage de corps de seringues, comprenant des logements de réception desdits corps de seringues.

La présente invention vise à remédier à ces inconvénients.

Son objectif principal est donc de fournir un ensemble d'éléments pour le groupage de corps de seringue, incluant un plateau de groupage qui présente une rigidité importante, de manière à assurer un parfait maintien des objets à grouper, sans conséquences néfastes sur les manipulations automatisées ou manuelles du plateau et sans limitation gênante en ce qui concerne les dimensions qu'il est possible de donner à ce plateau.

Un autre objectif de l'invention est de fournir un ensemble d'éléments permettant d'immobiliser les corps de seringue, par rapport au plateau de groupage, tant dans le sens de leur axe longitudinal qu'en pivotement autour de cet axe.

Un objectif supplémentaire de l'invention est de fournir un ensemble d'éléments permettant la mise en place sur les corps de seringue, avant remplissage, de pièces proximales de prise d'appui telles que précitées, tout en assurant une parfaite protection de ces pièces proximales de prise d'appui lors du transport des corps de seringue.

L'ensemble d'éléments concerné comprend, de manière connue en soi, un plateau à logements de réception des objets à grouper, chaque logement étant délimité par des parois latérales et comprenant une zone d'appui pour recevoir l'objet à grouper.

Le fait que les parois latérales soient attenantes permet à ces parois de constituer des lignes de rigidification continues du plateau, conférant à ce dernier une rigidité bien adaptée à des manipulations du plateau entre autres par des moyens automatisés. La réception des parties proximales des objets à grouper dans les alvéoles permet une parfaite protection de ces parties proximales, et le fait d'immobiliser les objets à grouper par rapport au plateau permet d'empêcher tout risque de détérioration d'un objet par un autre malgré la contiguïté des alvéoles.

L'ensemble d'éléments est utilisé pour le groupage de corps de seringue et comprend alors, outre ledit plateau, des pièces de prise d'appui destinées à être mises en place sur les extrémités proximales des corps de seringue afin de former des surfaces pour la prise d'appui des doigts de l'utilisateur au moment de la réalisation d'une injection, chaque alvéole présentant des dimensions correspondant au moins partiellement à celles d'une de ces pièces de prise d'appui, et chaque pièce de prise d'appui étant conformée pour coopérer avec le ou les moyens d'immobilisation que comprend chaque alvéole.

L'ensemble d'éléments permet ainsi un assemblage du plateau et de ces pièces de prise d'appui, et l'engagement d'une pièce de prise d'appui dans une alvéole est réalisé de manière précise.

Les parois latérales délimitant les alvéoles peuvent être légèrement évasées pour former une entrée d'alvéole ayant des dimensions supérieures à celles d'une pièce de prise d'appui.

Ces entrées d'alvéoles permettent de guider chaque pièce de prise d'appui lors de son engagement dans une alvéole.

Chaque pièce de prise d'appui peut être maintenue sur un corps de seringue avant la mise en place de ce corps de seringue dans une alvéole du plateau. Chaque pièce de prise d'appui peut également être conformée de manière à pouvoir être mise en place dans une alvéole du plateau et à pouvoir recevoir le corps de seringue après cette mise en place.

Selon une forme de réalisation possible de chaque pièce de prise d'appui dans ce deuxième cas, chaque pièce de prise d'appui comprend une ouverture pour l'engagement d'un corps de seringue et une série de doigts disposés radialement dans cette ouverture, ces doigts étant conformés pour venir serrer le corps de seringue entre eux, avec flexion, lorsque ce corps de seringue est engagé dans ladite ouverture, afin de réaliser l'assemblage de la pièce de prise d'appui et du corps de seringue.

Chaque moyen d'immobilisation est constitué par au moins une dent d'encliquetage aménagée en une zone d'une paroi latérale délimitant l'alvéole.

Ces dents permettent de réaliser l'immobilisation des corps de seringue à grouper par suite de la simple insertion de ces corps de seringue dans les alvéoles, tout en réalisant une immobilisation fiable.

Selon une forme de réalisation possible de l'invention dans ce cas, une paroi délimitant deux alvéoles adjacentes comprend un évidement aménagé dans son épaisseur, à partir de la tranche de cette paroi, cet évidement individualisant deux dents d'encliquetage, une pour chacune de ces alvéoles.

Selon une autre forme de réalisation possible de l'invention dans ce même cas, une paroi délimitant deux alvéoles adjacentes comprend une encoche dans laquelle est située une dent d'encliquetage unique, cette dent d'encliquetage comprenant deux saillies d'encliquetage sur ses deux faces opposées, une pour chacune de ces alvéoles.

Chaque alvéole présente de préférence une forme carrée ou rectangulaire, de sorte que les parois latérales qui délimitent les alvéoles constituent des lignes de rigidification perpendiculaires entre elles, conférant au plateau une rigidité importante dans les directions de ces lignes de rigidification.

Chaque alvéole comprend alors avantageusement deux moyens d'immobilisation disposés sur deux côtés opposés de l'alvéole, notamment, lorsque l'alvéole est rectangulaire, sur les petits côtés de cette alvéole rectangulaire.

Ces deux moyens d'immobilisation mutuellement distants assurent un parfait maintien du corps de seringue par rapport au plateau.

Chaque zone d'appui est constituée par au moins une paroi formant un rebord s'étendant à l'intérieur de l'alvéole.

Le plateau peut comprendre des zones permettant ou favorisant sa préhension. Il peut notamment s'agir de parois s'étendant sensiblement dans le plan du plateau, ces parois formant des surfaces sur lesquelles peuvent venir s'appliquer des ventouses à dépression équipant une machine de manipulation du plateau.

De préférence, le plateau est moulé en une seule pièce de matière synthétique.

Pour sa bonne compréhension, l'invention est à nouveau décrite ci-dessous en référence au dessin schématique annexé représentant, à titre d'exemples non limitatifs, deux formes de réalisation possibles de l'ensemble d'éléments qu'elle concerne.
La figure 1 est une vue partielle, en perspective, d'une boîte d'emballage de corps de seringue dans laquelle est placé un plateau de groupage faisant partie de cet ensemble d'éléments, selon la première forme de réalisation ;
la figure 2 est une vue en perspective, à échelle agrandie, de deux alvéoles du plateau de groupage et d'une pièce proximale d'appui placée dans une de ces alvéoles, faisant également partie dudit ensemble d'éléments, cette pièce proximale d'appui équipant l'extrémité proximale d'un corps de seringue ;
la figure 3 est une vue de la pièce proximale d'appui et de l'alvéole recevant cette pièce, en coupe selon la ligne III-III de la figure 2 ;
la figure 4 est une vue en perspective, à échelle agrandie, de trois alvéoles du plateau de groupage et d'une pièce proximale d'appui placée dans une de ces alvéoles, selon une deuxième forme de réalisation, et
la figure 5 est une vue de la pièce proximale d'appui et de l'alvéole recevant cette pièce, en coupe selon la ligne V-V de la figure 4, au cours de l'engagement d'un corps de seringue au travers de ladite pièce.

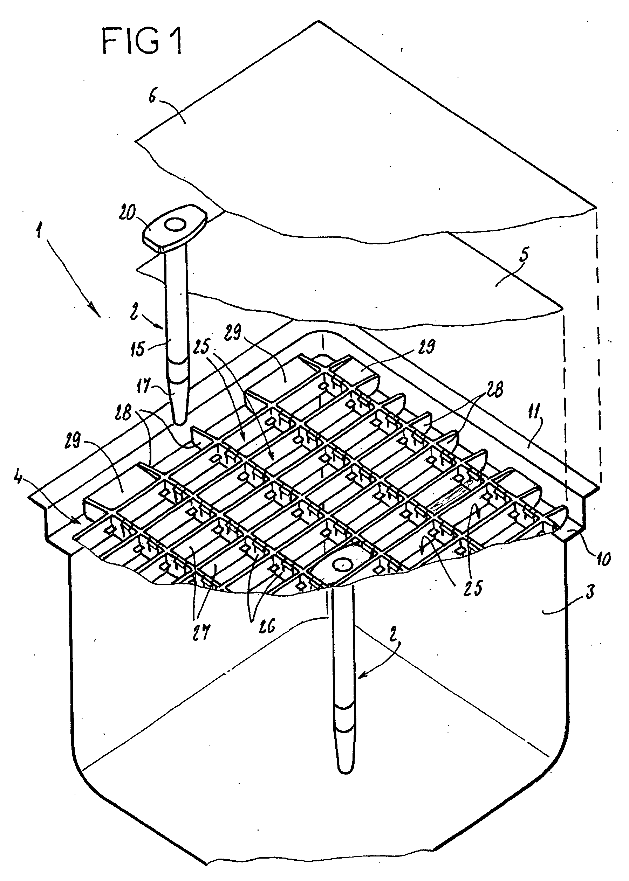

La figure 1 représente un emballage 1 utilisé pour le transport de corps de seringue 2 d'un site de fabrication de ces corps de seringue 2 à un site de traitement ultérieur de ces corps de seringue 2, notamment de remplissage de ceux-ci.

L'emballage 1 comprend une boîte 3, un plateau 4 de groupage des corps de seringue 2, une feuille de protection 5 posée sur le dessus du plateau 4 et une feuille de scellage 6 fermant la boîte 3 hermétiquement.

La boîte 3 est en matière synthétique. Elle comprend un épaulement supérieur 10 de réception des bords du plateau 4 et un rebord extérieur 11 permettant le scellage de la feuille 6.

Le plateau 4 sera décrit plus loin en détail.

La feuille de protection 5 permet de protéger les corps de seringue 2 lors d'opérations ultérieures telles que stérilisation, transport et décontamination de la boîte 3. Elle peut notamment être en matériau connu sous la dénomination "TYVEK® ", fabriqué par la société Dupont de Nemours.

La feuille de scellage 6 assure la fermeture hermétique de la boîte 3. Elle peut également être en "TYVEK® ".

Ces feuilles 5, 6 sont perméables aux gaz ou rayonnements de stérilisation mais étanches aux bactéries.

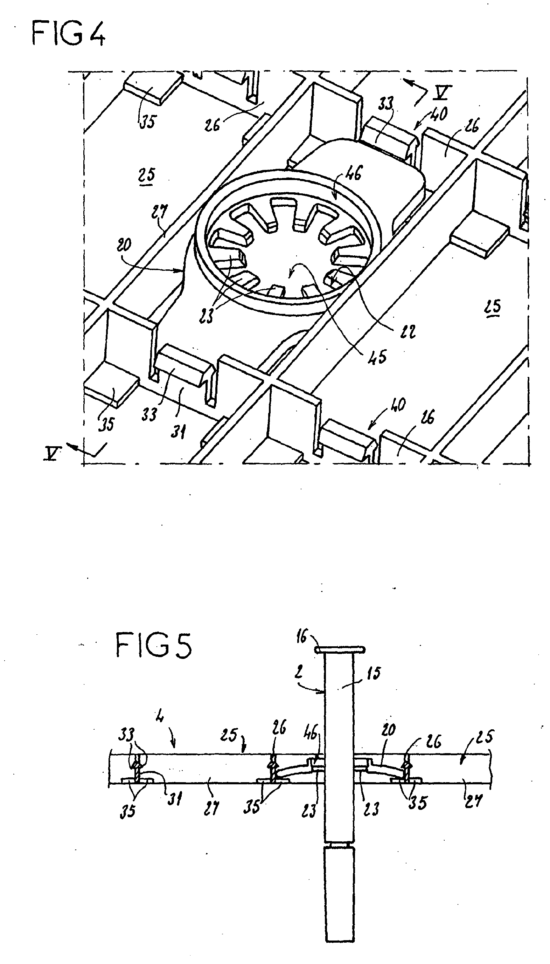

Les corps de seringue 2 comprennent classiquement un corps creux 15 se terminant, à une extrémité distale, par un embout comportant, ou propre à recevoir, une aiguille d'injection, et une collerette proximale 16. Dans l'exemple représenté sur la figure 1, l'extrémité distale de chaque corps creux 15 comporte un capuchon 17 de protection de l'embout et de l'éventuelle aiguille, tandis que l'extrémité proximale de ce même corps 15 reçoit, par encliquetage sur ladite collerette, une pièce proximale d'appui 20. Cette pièce proximale 20 permet l'appui des doigts de l'utilisateur de part et d'autre du corps 15 au moment de la réalisation d'une injection. Dans l'exemple représenté sur la figure 5, le corps de seringue 2 est engagé au travers d'une ouverture 22 aménagée dans la pièce proximale d'appui 20 jusqu'à venue de la collerette proximale 16 de ce corps de seringue 2 en appui contre des doigts 23 que comprend la pièce 20, ainsi que cela sera décrit plus en détail plus loin.

Le plateau 4 est réalisé par moulage d'une matière synthétique en une seule pièce. Comme cela apparaît sur la figure 1, il délimite une pluralité d'alvéoles rectangulaires 25 disposées côte-à-côte, délimitées par des parois rectilignes 26, 27, ces parois 26, 27 se croisant à angle droit et s'étendant en largeur selon une direction perpendiculaire au plan général du plateau 4. Chaque paroi 26, 27 se prolonge, au niveau des bords du plateau 4, au-delà de la paroi perpendiculaire 27, 26 la plus proche, pour former des pattes 28 de prise d'appui sur l'épaulement 10 de la boîte 3. En certains emplacements, deux pattes 28 consécutives sont reliées l'une à l'autre par une paroi 29 s'étendant dans le plan général du plateau 4, dont les faces sont lisses. Ces parois 29 permettent l'adhésion du plateau 4 à des ventouses à dépression équipant des moyens de manipulation automatiques du plateau 4, ces moyens de manipulation permettant de saisir le plateau 4 en vue de l'engagement de celui-ci dans la boîte 3 et/ou de son extraction hors de cette boîte 3 après transport, et/ou du transfert de ce plateau 4 vers des unités subséquentes de traitement des corps de seringue 2, notamment des unités de remplissage de ces corps de seringue 2.

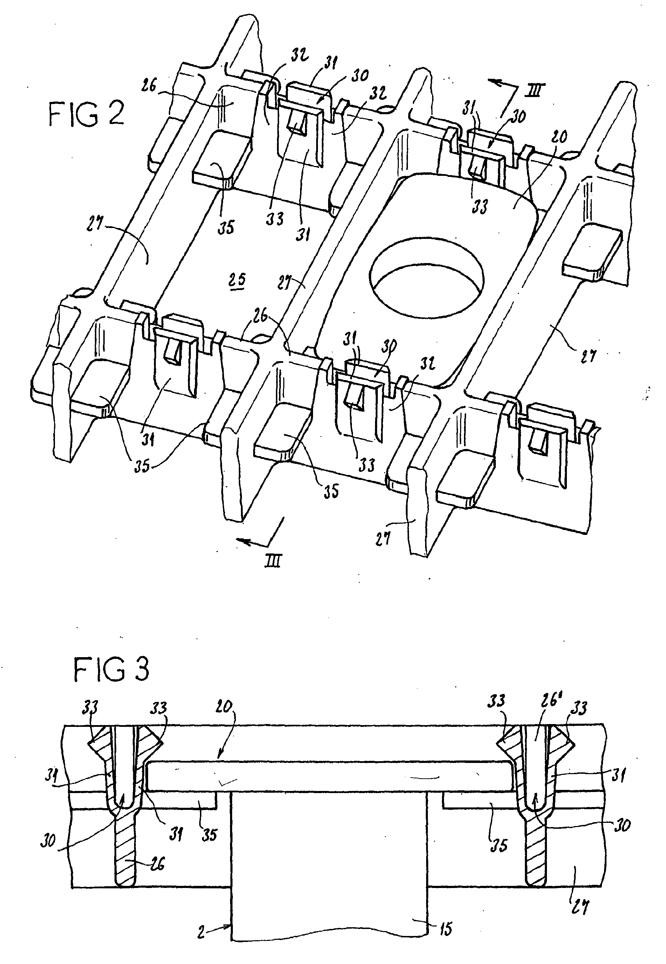

En référence aux figures 2 et 3, l'on retrouve deux alvéoles 25 du plateau 4 et un corps de seringue 2 dont la pièce proximale d'appui 20 est engagée dans une alvéole 25.

Comme cela est visible sur ces figures, chaque partie de paroi 26 délimitant un petit côté d'une alvéole 25 présente, dans la zone médiane de ce petit côté, un évidement 30 aménagé dans l'épaisseur de cette partie de paroi 26, à partir de la tranche de cette paroi. Cet évidement 30 délimite deux dents d'encliquetage 31 reliées aux bords contigus de la paroi 26 par des voiles 32.

Chaque dent d'encliquetage 31 est reliée à la paroi 26 au niveau de sa partie inférieure et comprend, sur sa face extérieure par rapport à l'évidement 30, une saillie d'encliquetage 33 de forme triangulaire. Cette saillie 33 délimite un bord supérieur incliné, qui forme une rampe contre laquelle la pièce 20 appuie lors de son engagement dans l'alvéole 25, et un bord inférieur de rétention de la pièce 20 dans l'alvéole 25. Il se comprend aisément en référence à la figure 3 que l'appui de la pièce 20 contre les deux saillies 33 en regard d'une même alvéole 25 provoque un effacement des deux dents 31 vers l'intérieur des évidements 30 afin d'autoriser le passage de la pièce 20 au-delà des saillies 33, les voiles 32 autorisant cet effacement de par leur souplesse ; une fois que la pièce 20 a franchi les saillies 33, les dents 31 retrouvent leur forme normale montrée sur la figure 3, dans laquelle les saillies 33 retiennent la pièce 20 dans l'alvéole 25.

Chaque alvéole 25 comprend en outre quatre parois 35 situées au niveau de ses angles, formant des appuis de réception d'une pièce 20. Comme le montre plus particulièrement la figure 3, les faces de ces parois 35 tournées du côté des saillies 33 sont situées à une distance de la zone inférieure de ces saillies 33 correspondant sensiblement à l'épaisseur des parties latérales d'une pièce 20. Les dents 31 maintiennent ainsi la pièce 20 plaquée contre les parois 35, et assurent donc une immobilisation du corps de seringue 2 par rapport au plateau 4.

Les figures 4 et 5 montrent un plateau 4 et une pièce 20 très similaires à ceux qui viennent d'être décrits. Par simplification, les éléments déjà décrits en référence aux figures 1 à 3, qui se retrouvent dans ce plateau 4 et cette pièce 20, ne seront pas à nouveau décrits et sont désignés par les mêmes références numériques.

Dans ce cas, chaque paroi 26 comprend une encoche 40 dans laquelle est située une dent d'encliquetage 31 unique, cette dent 31 comprenant des saillies d'encliquetage 33 sur ses deux faces opposées. Une même dent d'encliquetage 31 permet la rétention de deux pièces 20 dans deux alvéoles 25 consécutives dans le sens longitudinal de ces alvéoles.

Chaque pièce proximale d'appui 20 comprend quant à elle l'ouverture 22 et les doigts 23 précités. Ces doigts 23 font saillie radialement vers le centre de cette ouverture 22, et leurs extrémités libres définissent un passage circulaire central 45 de diamètre légèrement inférieur au diamètre du corps creux 15 d'un corps de seringue 2. Comme cela se comprend en référence à la figure 5, la pièce 20 peut être mise en place dans une alvéole 25 par encliquetage de la manière précitée puis un corps de seringue 2 peut être introduit dans ledit passage 45 jusqu'à venue de la collerette proximale 16 du corps 15 au contact des doigts 23 et dans un évidement 46 que comprend la pièce 20 à cet effet. Lors de cette introduction, les doigts 23 fléchissent et viennent serrer le corps 15 entre eux, ce qui réalise l'assemblage de la pièce de prise d'appui 20 et du corps de seringue 2.

Il apparaît de ce qui précède que l'invention apporte une amélioration déterminante à la technique antérieure, en fournissant un ensemble d'éléments pour le groupage d'objets allongés, notamment de corps de seringue 2, qui inclut un plateau 4 à rigidité bien adaptée à des manipulations par des moyens automatisés et n'ayant pas de limitations gênantes quant à ses dimensions.

Cet ensemble d'éléments permet d'immobiliser les corps de seringue 2 par rapport au plateau 4, tant dans le sens de l'axe longitudinal de ces corps de seringue 2 qu'en pivotement de ceux-ci autour de cet axe.

L'ensemble d'éléments permet en outre la mise en place sur les corps de seringue 2, avant remplissage, de pièces proximales de prise d'appui 20, tout en assurant une parfaite protection de ces pièces 20 lors du transport des corps de seringue.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées. Ainsi, il est clair que la pièce 20 montrée sur la figure 4 peut être utilisée avec un plateau 4 tel que montré sur la figure 2, et qu'une pièce 20 telle que montrée sur la figure 2 peut être utilisée avec un plateau 4 tel que montré sur la figure 4 ; chaque dent d'encliquetage 31 montrée sur la figure 4, comprenant des saillies d'encliquetage 33 sur ses deux faces opposées, pourrait être remplacée par une paires de dents ayant chacune une saillie 33 sur l'une de ses faces, les saillie 33 de ces deux dents 31 étant situées sur deux faces opposées de ces dents 31 ; la forme des alvéoles peut être autre que rectangulaire, notamment carrée, hexagonale, octogonale, ou polygonale d'une manière générale.

## Revendications

1. Ensemble d'éléments pour le groupage de corps de seringue (2), comprenant un plateau (4) à logements (25) de réception des corps de seringue (2) à grouper, chaque logement (25) étant délimité par des parois latérales (26, 27) et comprenant une zone d'appui (35) pour recevoir le corps de seringue à grouper ; de telle sorte à ce que les corps de seringue à grouper s'étendent dans une direction sensiblement perpendiculaire au plan du plateau
ensemble d'éléments **caractérisé en ce que** :
- chaque logement est formé par une alvéole (25) ;
- les parois latérales (26, 27) délimitant les alvéoles (25) sont attenantes les unes aux autres d'une alvéole à une alvéole adjacente et s'étendent dans une direction sensiblement perpendiculaire au plan du plateau (4) ;
- chaque alvéole (25) est conformée pour recevoir en elle au moins partiellement la partie proximale (16, 20) d'un corps de seringue à grouper,
- chaque alvéole (25) comprend au moins un moyen (31) d'immobilisation de chaque corps de seringue à grouper, ledit ensemble d'éléments comprenant en outre des pièces (20) de prise d'appui destinées à être mises en place sur les extrémités proximales des corps de seringue (2) afin de former des surfaces pour la prise d'appui des doigts de l'utilisateur au moment de la réalisation d'une injection, chaque alvéole (25) présentant des dimensions correspondant à celles d'une de ces pièces de prise d'appui (20), et chaque pièce de prise d'appui (20) étant conformée pour coopérer avec le ou les moyens d'immobilisation (31) que comprend chaque alvéole (25), chaque moyen d'immobilisation étant constitué par au moins une dent d'encliquetage (31) aménagée en une zone d'une paroi latérale (26) délimitant l'alvéole (25), chaque zone d'appui étant constituée par au moins une paroi (35) formant un rebord s'étendant à l'intérieur de l'alvéole (25), la ou les dents (31) maintenant la pièce d'appui (20) contre la ou les parois (35).

2. Ensemble selon la revendication 1, **caractérisé en ce que** les parois latérales (26, 27) délimitant les alvéoles (25) sont légèrement évasées pour former une entrée d'alvéole (25) ayant des dimensions supérieures à celles d'une pièce de prise d'appui (20).

3. Ensemble selon la revendication 2, **caractérisé en ce que** chaque pièce de prise d'appui (20) est conformée de manière à pouvoir être mise en place dans une alvéole (25) du plateau (4) et à pouvoir recevoir le corps de seringue (2) après cette mise en place.

4. Ensemble selon la revendication 3, **caractérisé en ce que** chaque pièce de prise d'appui (20) comprend une ouverture (22) pour l'engagement d'un corps de seringue (2) et une série de doigts (23) disposés radialement dans cette ouverture (22), ces doigts (23) étant conformés pour venir serrer le corps de seringue (2) entre eux, avec flexion, lorsque ce corps de seringue (2) est engagé dans ladite ouverture (22), afin de réaliser l'assemblage de la pièce de prise d'appui (20) et du corps de seringue (2).

5. Ensemble selon la revendication 1, **caractérisé en ce qu'**une paroi (26) délimitant deux alvéoles (25) adjacentes comprend un évidement (30) aménagé dans son épaisseur, à partir de la tranche de cette paroi (26), cet évidement (30) individualisant deux dents d'encliquetage (31), une pour chacune de ces alvéoles (25).

6. Ensemble selon la revendication 1, **caractérisé en ce qu'**une paroi (26) délimitant deux alvéoles (25) adjacentes comprend une encoche (40) dans laquelle est située une dent d'encliquetage (31) unique, cette dent d'encliquetage (31) comprenant deux saillies d'encliquetage (33) sur ses deux faces opposés, une pour chacune de ces alvéoles (25).

7. Ensemble selon l'une des revendications 1 à 6, **caractérisé en ce que** chaque alvéole (25) présente une forme carrée ou rectangulaire.

8. Ensemble selon la revendication 7, **caractérisé en ce que** chaque alvéole (25) comprend deux moyens d'immobilisation (31) disposés sur deux côtés opposés de l'alvéole (25).

9. Ensemble selon la revendication 8, **caractérisé en ce que** chaque alvéole (25) est rectangulaire et **en ce que** les deux moyens d'immobilisation (31) sont disposés sur les petits côtés de l'alvéole (25).

10. Ensemble selon l'une des revendications 1 à 9, **caractérisé en ce que** le plateau (4) comprend des zones (29) permettant ou favorisant sa préhension,
lesdites zones permettant ou favorisant la préhension du plateau (4) étant constituées par des parois (29) s'étendant sensiblement dans le plan du plateau (4), ces parois (29) formant des surfaces sur lesquelles peuvent venir s'appliquer des ventouses à dépression équipant une machine de manipulation du plateau (4).

11. Ensemble selon l'une des revendications 1 à 10, **caractérisé en ce que** le plateau (4) est moulé en une seule pièce de matière synthétique.

## Claims

1. An assembly of elements for bulking syringe bodies (2), comprising a plate (4) with housings (25) for receiving syringe bodies (2) for bulking, each housing (25) being delimited by lateral walls (26, 27) and comprising a support zone (35) for receiving the syringe body for bulking, so that syringe bodies for bulking extend in a direction substantially perpendicular to the plane of the plate,
assembly of elements wherein:
- each housing is formed by a cell (25);
- the lateral walls (26, 27) delimiting the cells (25) adjoin one another from one cell to an adjacent cell and extend in a direction substantially perpendicular to the plane of the plate (4);
- each cell (25) is shaped in order to at least partially receive in it the proximal part (16, 20) of a syringe body for bulking; and
- each cell (25) comprises at least one means (31) for immobilizing each syringe body for bulking, said assembly of elements further comprising support hold pieces (20) for placing on the proximal ends of the syringe bodies (2), in order to form surfaces for the support holding of the user's fingers when carrying out an injection, each cell (25) having dimensions that correspond to those of one of these support hold pieces (20) and each support hold piece (20) being shaped in order to interact with the immobilization means (31) of each cell, each immobilization means consisting of at least one snap-fitting tooth (31) provided in a zone of a lateral wall (26) delimiting the cell (25), each support zone consisting of at least one wall (35) forming a rim extending inside the cell (25), the snap-fitting tooth (31) maintaining the support hold piece (20) against said wall (35).

2. The assembly as claimed in claim 1, wherein the lateral walls (26, 27) delimiting the cells (25) are slightly flared in order to form a cell (25) entry having dimensions greater than those of a support hold piece (20).

3. The assembly as claimed in claim 2, wherein each support hold piece (20) is shaped so that it is possible for it to be placed in a cell (25) of the plate (4) and for it to receive the syringe body (2) after said placing.

4. The assembly as claimed in claim 3, wherein each support hold piece (20) comprises an opening (22) for the engagement of a syringe body (2) and a series of fingers (23) arranged radially in this opening (22), these fingers (23) being shaped in order to grip the syringe body (2) between them, with flexing, when this syringe body (2) is engaged in said opening (22), in order to assemble the support hold piece (20) and the syringe body (2).

5. The assembly as claimed in claim 1, wherein a wall (26) delimiting two adjacent cells (25) comprises a recess (30) provided in its thickness, from the section of this wall (26), this recess (30) forming two separate snap-fitting teeth (31), one for each of these cells (25).

6. The assembly as claimed in claim 1, wherein a wall (26) delimiting two adjacent cells (25) comprises a notch (40) in which there is a single snap-fitting tooth (31), this snap-fitting tooth (31) comprising two snap-fitting projections (33) on its two opposite faces, one for each of these cells (25).

7. The assembly as claimed in one of claims 1 to 6, wherein each cell (25) has a square or rectangular shape.

8. The assembly as claimed in claim 7, wherein each cell (25) comprises two immobilization means (31) arranged on two opposite sides of the cell (25).

9. The assembly as claimed in claim 8, wherein each cell (25) is rectangular and wherein the two immobilization means (31) are arranged on the smaller sides of the cell (25).

10. The assembly as claimed in one of claims 1 to 9, wherein the plate (4) comprises zones (29) allowing or promoting its gripping, said zones allowing or promoting the gripping of the plate (4) consisting of walls (29) extending substantially in the plane of the plate (4), these walls (29) forming surfaces on which vacuum suction cups equipping a machine for handling the plate (4) may be applied.

11. The assembly as claimed in one of claims 1 to 10, wherein the plate (4) is molded as a single piece of synthetic material.

## Patentansprüche

1. Elementegarnitur zum Gruppieren von Spritzenkörpern (2), die eine Platte (4) mit Einlassungen (25) zur Aufnahme der zu gruppierenden Spritzenkörper (2) umfasst, wobei jede Einlassung (25) von Seitenwänden (26, 27) begrenzt ist und eine Abstützzone (35) zur Aufnahme des zu gruppierenden Spritzenkörpers derart umfasst, dass sich die zu gruppierenden Spritzenkörper in eine zur Ebene der Platte etwa senkrechte Richtung erstrecken,
wobei die Elementegarnitur **dadurch gekennzeichnet ist, dass**:
- jede Einlassung von einer Zelle (25) gebildet wird,
- die die Zellen (25) begrenzenden Seitenwände (26, 27) einer Zelle an eine benachbarte Zelle angrenzen und sich in eine zur Ebene der Platte (4) etwa senkrechte Richtung erstrecken,
- jede Zelle (25) ausgebildet ist, um in ihr mindestens teilweise den proximalen Abschnitt (16, 20) eines zu gruppierenden Spritzenkörpers aufzunehmen,
- jede Zelle (25) mindestens ein Mittel (31) zum Blockieren jedes zu gruppierenden Spritzenkörpers umfasst, wobei die Elementegarnitur weiterhin Abstützteile (20) umfasst, die dazu bestimmt sind, auf den proximalen Enden der Spritzenkörper (2) platziert zu sein, um Flächen zur Abstützung der Finger des Benutzers im Augenblick der Durchführung einer Injektion zu bilden, wobei jede Zelle (25) Abmessungen aufweist, die denen einer dieser Abstützteile (20) entsprechen, und jedes Abstützteil (20) ausgebildet ist, um mit dem oder den Blockiermitteln (31), die jede Zelle (25) umfasst, zusammenzuarbeiten, wobei jedes Blockiermittel von mindestens einem Rastzahn (31) gebildet wird, der in eine eine Zelle (25) begrenzende Zone einer Seitenwand (26) eingearbeitet ist, wobei jeder Abstützbereich von mindestens einer Wand (35) gebildet wird, die einen Randanschlag bildet, der sich innerhalb der Zelle (25) erstreckt, wobei der oder die Zähne (31) das Abstützteil (31) gegen die Wand oder Wände (25) halten.

2. Garnitur nach Anspruch 1, **dadurch gekennzeichnet, dass** die die Zellen begrenzenden (25) Seitenwände (26, 27) leicht erweitert sind, um einen Zelleneingang (25) mit Abmessungen zu bilden, die größer sind als die eines Abstützteils (20).

3. Garnitur nach Anspruch 2, **dadurch gekennzeichnet, dass** jedes Abstützteil (20) derart ausgebildet ist, dass es in eine Zelle (25) der Platte (4) platzierbar ist und den Spritzenkörper (2) nach dieser Platzierung aufnehmen kann.

4. Garnitur nach Anspruch 3, **dadurch gekennzeichnet, dass** jedes Abstützteil (20) eine Öffnung (22) zum Einsetzen eines Spritzenkörpers (2) umfasst und eine Reihe von radial in dieser Öffnung (22) angeordneten Fingern (23), wobei diese Finger (23) ausgebildet sind, um den Spritzenkörper (2) mit Biegung zwischen sich einzuspannen, wenn dieser Spritzenkörper (2) in die Öffnung (22) eingesetzt ist, um die Montage des Abstützteils (2) und des Spritzenkörpers (2) durchzuführen.

5. Garnitur nach Anspruch 1, **dadurch gekennzeichnet, dass** eine zwei benachbarte Zellen (25) begrenzende Wand (26) eine ausgehend von der Schmalseite dieser Wand (26) in ihre Dicke eingearbeitete Aussparung (30) umfasst, wobei diese Aussparung (30) zwei Rastzähne (31) - einen für jede dieser Zellen (25) - individualisiert.

6. Garnitur nach Anspruch 1, **dadurch gekennzeichnet, dass** eine zwei benachbarte Zellen (25) begrenzende Wand (26) einen Schlitz (40) umfasst, in dem sich ein einziger Rastzahn (31) befindet, wobei dieser Rastzahn (31) auf seinen zwei gegenüberliegenden Seiten zwei Rastvorsprünge (33) - einen für jede dieser Zellen (25) - umfasst.

7. Garnitur nach einem der Ansprüche 1 bis 6, **dadurch gekennzeichnet, dass** jede Zelle (25) eine quadratische oder rechteckige Form aufweist.

8. Garnitur nach Anspruch 7, **dadurch gekennzeichnet, dass** jede Zelle (25) zwei Blockiermittel (31) umfasst, die auf zwei gegenüberliegenden Seiten der Zelle (25) angeordnet sind.

9. Garnitur nach Anspruch 8, **dadurch gekennzeichnet, dass** jede Zelle (25) rechteckig ist und dass die zwei Blockiermittel (31) auf den kleinen Seiten der Zelle (25) angeordnet sind.

10. Garnitur nach einem der Ansprüche 1 bis 9, **dadurch gekennzeichnet, dass** die Platte (4) Zonen (29) umfasst, die ihr Greifen erlauben oder fördern,
wobei die Zonen, die das Greifen der Platte (4) erlauben oder fördern, von Wänden (29) gebildet sind, die sich etwa in der Ebene der Platte (4) erstrecken, wobei diese Wände (29) Flächen bilden, auf denen Unterdrucksaugnäpfe anhaften können, die eine Maschine zur Handhabung der Platte (4) ausrüsten.

11. Garnitur nach einem der Ansprüche 1 bis 10, **dadurch gekennzeichnet, dass** die Platte (4) in einem einzigen Stück aus Kunststoff geformt ist.
